# EUROPÄISCHE PATENTSCHRIFT

(11) **EP 0 835 656 B1**
(45) Veröffentlichungstag und Bekanntmachung des Hinweises auf die Patenterteilung: **28.11.2001**
(21) Anmeldenummer: 97116122.9
(22) Anmeldetag: 17.09.1997
(51) Int. Cl.: A61K 31/135, A61K 31/44, A61K 31/55, A61K 31/495

(54) **Kombinationspräparat enthaltend Tramadol und einen Calcium-Kanal Antagonisten**
Combination drug containing tramadol and a calcium channel antagonist
Préparations combinées contenant du tramadol et un antagoniste des canaux à calcium

(30) Priorität: 09.10.1996 DE 19641576
(43) Veröffentlichungstag der Anmeldung: 15.04.1998
(73) Patentinhaber: Grünenthal GmbH, 52078 Aachen (DE)
(72) Erfinder: Reimann, Wolfgang Siegfried, Dr., 52078 Aachen (DE)

(56) Entgegenhaltungen:
- EP-A- 0 546 676
- WO-A-93/04675
- POZO DEL E ET AL: "ANALGESIC EFFECTS OF SEVERAL CALCIUM CHANNEL BLOCKERS IN MICE" EUROPEAN JOURNAL OF PHARMACOLOGY, Bd. 137, 1987, Seiten 155-160, XP000603056
- CONTRERAS E ET AL: "CALCIUM CHANNEL ANTAGONISTS INCREASE MORPHINE-INDUCED ANALGESIA AND ANTAGONIZE MORPHINE TOLERANCE" EUROPEAN JOURNAL OF PHARMACOLOGY, Bd. 148, 1988, Seiten 463-466, XP000603055
- BORMANN V. B. ET AL: "Additive Analgesie durch Calcium-antagonisten" FORTSCHRITTE DER ANÄSTHESIOLOGIE. NOTFALL- UND INTENSIVMEDIZIN, Nr. 1, September 1987, Seiten 5-10, XP002052761

## Beschreibung

Die vorliegende Erfindung bezieht sich auf ein Arzneimittel insbesondere zur Behandlung von Schmerzen, das eine Kombination des Schmerzmittels Tramadol mit einem Calcium-Kanal Antagonisten enthalt.

Opioide werden aufgrund ihrer starken analgetischen Wirkung zur Linderung mittelschwerer bis schwerster akuter Schmerzen eingesetzt. Ein großer Nachteil bei der Verwendung von Opioiden liegt jedoch in den damit verbundenen starken Nebenwirkungen. So treten häufig Wirkungen auf den Gastrointestinaltrakt wie Obstipation auf, weiterhin stellen sich Atemdepression sowie bei wiederholter Gabe eine Abhängigkeit ein, die zu Mißbrauch fuhren kann. Weiterhin ist die Toleranzentwicklung ungunstig.

Seit mehreren Jahren ist bekannt, daß der analgetische Effekt von Opioiden durch gleichzeitiges Verabreichen von organischen Calcium-Kanal Antagonisten verstarkt werden kann (Fortschr. Anaesth. 1987, 5). Calcium-Kanal Antagonisten lassen sich in solche des Typs der Dihydropyridine, der Benzothiazepine sowie der Phenylalkylamine einteilen. Sie werden üblicherweise zur Behandlung kardiovasculärer Krankheitszustände wie Bluthochdruck, Arhythmie oder Angina Pectoris eingesetzt. Die Wirkungsweise dieser Substanzen beruht auf der selektiven Unterdrückung des Ca²⁺-Flusses in den Ca²⁻-Kanalen des Herzens und des peripheren Vascularsystems. Calcium-Kanale mit einer hohen Affinitat für Calcium-Kanal Antagonisten wurden ebenfalls im Gehirn nachgewiesen, so daß auch eine zentrale Wirkung der Calcium-Kanal-Antagonisten wahrscheinlich erscheint.

Durch die Verstarkung der antinozizeptiven Wirkung der Opioide mittels Calcium-Kanal Antagonisten können niedrigere Dosen des Opioids bei gleicher analgetischer Wirkung verabreicht werden. Hierdurch lassen sich die oben genannten Nebenwirkungen vermindern. Allerdings muß auch bei fortgesetzter Gabe trotz geringerer Opioidmengen mit dem Auftreten von Abhängigkeit, Atemdepression und Obstipation gerechnet werden. Wegen der hohen analgetischen Wirksamkeit von Opioiden kann jedoch bis heute nicht auf deren Einsatz bei der Behandlung von Schmerzen verzichtet werden.

Der vorliegenden Erfindung liegt daher die Aufgabe zugrunde, ein neues Arzneimittel zu entwickeln, das ein Analgetikum enthält, das mit einem Calcium-Kanal Antagonisten kombiniert und in niedrigen Dosen verabreicht werden kann und das die oben beschriebenen Nachteile von Opioiden nicht aufweist.

Es wurde gefunden, daß bei einer Kombinationstherapie von Tramadol bzw. seinen pharmazeutisch vertraglichen Salzen mit Calcium-Kanal Antagonisten die analgetische Wirkung von Tramadol verstärkt wird.

Gegenstand der Erfindung ist dementsprechend ein Kombinationspräparat, enthaltend Tramadol in Form der freien Base oder eines physiologisch verträglichen Salzes und mindestens einen Calcium-Kanal Antagonisten in getrennter oder gemeinsamer Formulierung.

Tramadol ist der INN-Name für das zentral wirkende Analgetikum (1RS;2RS)-2-[(Dimethylamino)methyl]-1-(3-methoxyphenyl-cyclohexanol). Es wird vorzugsweise in Form seines Hydrochlorids verabreicht. Üblicherweise wird ein Racemat aus (+)- und (-)-Tramadol eingesetzt. Es ist jedoch auch möglich, nur jeweils eines der beiden Enantiomeren zu verabreichen.

Die erfindungsgemaßen Kombinationspraparate bewirken keine signifikante Atemdepression und weisen ein niedriges Potential für die Entwicklung von Toleranz, Abhangigkeit und Mißbrauch auf. Das Wirkspektrum der Kombinationspraparate reicht bis in die Neuropathie.

Als Calcium-Kanal Antagonisten eignen sich solche des Dihydropyridin-, des Benzothiazepin- und des Phenylalkylamintyps. Beispiele für Antagonisten aus der Dihydropyridingruppe sind Nimodipin, Nicardipin und Nifedipin, für Antagonisten des Benzothiazepintyps Diltiazem und für solche des Phenylalkylamintyps Verapamil, Gallopamil, Flunarizin und Cinnarizin.

Vorzugsweise wird bei der Kombinationstherapie, die Gegenstand der vorliegenden Erfindung ist, Tramadol in Verbindung mit nur einem Calcium-Kanal Antagonisten eingesetzt. Es ist jedoch auch möglich, zwei oder mehrere dieser Antagonisten einzusetzen. Werden mehrere Calcium-Kanal Antagonisten eingesetzt, können diese aus nur einer oder aus verschiedenen der oben genannten Typklassen stammen.

Die Wirkstoffe der erfindungsgemäßen Kombinationspraparate können als gemeinsame Formulierung oder getrennt verabreicht werden.

Erfindungsgemäße Kombinationspräparate zur gemeinsamen Verabreichung der Wirkstoffe enthalten neben Tramadol in Form der freien Base oder eines physiologisch verträglichen Salzes und einem oder mehreren Calcium-Kanal Antagonisten üblicherweise Tragermaterialien, Füllstoffe, Lösungsmittel, Verdünnungsmittel, Farbstoffe und/oder Bindemittel. Die Auswahl der Hilfsstoffe sowie die einzusetzenden Mengen hangen davon ab, ob das erfindungsgemaße Arzneimittel oral, intravenös, intraperitoneal, intradermal, intramuskular, intranasal, buccal oder örtlich, zum Beispiel auf Infektionen an der Haut, an den Schleimhäuten oder an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Losungen, Suspensionen, leicht rekonstuierbare Trockenzubereitungen sowie Sprays. Erfindungsgemäße Kombinationspräparate in einem Depot in gelöster Form oder in einem Pflaster, gegebenenfalls unter Zusatz von die Hautpenetration fördernden Mitteln, sind Beispiele für geeignete perkutane Zubereitungsformen. Aus oral oder perkutan anwendbaren Zubereitungsformen können die Wirkstoffe verzögert freigesetzt werden.

Bei getrenntem Verabreichen der Wirkstoffe werden generell die üblichen zugelassenen, einem Fachmann bekannten Darreichungsformen für Tramadol und Calcium-Kanal Antagonisten verwendet. Bekannte Darreichungsformen fur Tramadol sind beispielsweise Tabletten, Retardtabletten, Tropfen, Kapseln, Suppositorien, Infusionslösungen und Injektionslösungen, insbesondere als Fertigspritze.

Calcium-Kanal-Antagonisten lassen sich beispielsweise als Injektions- oder Infusionslosung, Tropfen, Kapsel, Film- oder Retardtablette, Retardkapsel oder Dragée verabreichen. Bevorzugt werden Tabletten, Kapseln, Tropfen und Infusionslösungen.

Die an den Patienten zu verabreichenden Wirkstoffmengen variieren in Abhängigkeit vom Gewicht des Patienten, von der Applikationsart, der Indikation und dem Schweregrad der Erkrankung. Üblicherweise werden sowohl bei der getrennten als auch bei der gemeinsamen Formulierung als Tagesdosierung 10 - 800 mg, vorzugsweise 20 bis 400 mg Tramadol als Hydrochlorid oder der entsprechenden Menge eines anderen physiologisch vertraglichen Salzes oder der freien Base verabreicht. Die entsprechende Menge an Calcium-Kanal Antagonist reicht von einem Viertel der minimalen Wirkdosis bis zur maximalen Tagesdosis (im Beipackzettel angegeben), vorzugsweise von einem Viertel der minimalen Wirkdosis bis zur Hälfte der maximalen Tagesdosis.

Durch die Kombination der beiden Wirkstoffgruppen laßt sich die zur Unterdrückung von Schmerzen notwendige Menge an Tramadol deutlich vermindern. Ein erfindungsgemaßes Kombinationspräparat wird typischerweise zur Behandlung mittelschwerer bis schwerer akuter oder chronischer Schmerzen eingesetzt.

### Beispiele

### Beispiel 1

Es wurde die antinozizeptive Wirkung von Tramadol (als Hydrochlorid) und Calcium-Kanal Antagonisten einzeln und in gemeinsamer Losung untersucht. Dazu wurden die jeweiligen Wirkstoffe in Lösung Ratten intrathekal (rückenmarksnah) injiziert und 10 Minuten nach Injektion ein Tail-Flick-Test durchgeführt. Zur Simulation eines Schmerzreizes wurde ein gebündelter Lichtstrahl auf den Rattenschwanz gerichtet. Die aufgeführten Ergebnisse stellen die Latenzzeit bis zum Wegziehen des Schwanzes in % des maximal erreichbaren Werts (MPE)dar. Die Anzahl der Tiere pro Gruppe n betrug 10 (n = 10). Die angewandte Methodik ist bei Reimann et al. in Naunyn-Schmiedeberg's Arch Pharmacol. 350, 380 (1994) beschrieben.

| **Substanz** | **% MPE** |
|---|---|
| Tramadol 6 µg | 9,1 ± 3,2 |
| Tramadol 12 µg | 15,3 ± 6,3 |
| Nimodipin 10 µg | 17,3 ± 8,2 |
| Nimodipin 10 µg + Tramadol 6 µg | 55,6 ± 9,4 |
| Nimodipin 10 µg + Tramadol 12 µg | 70,6 ± 8,8 |
| Diltiazem 10 µg | 4,3 ± 5,1 |
| Diltiazem 10 µg + Tramadol 6 µg | 51,9 ± 6,4 |
| Diltiazem 10 µg + Tramadol 12 µg | 64,4 ± 8,6 |
| Verapamil 20 µg | 13,9 ± 9,3 |
| Verapamil 20 µg + Tramadol 6 µg | 31,5 ± 9,9 |
| Verapamil 20 µg + Tramadol 12 µg | 39,2 ± 9,3 |

Die Ergebnisse zeigen, daß die gleichzeitige Verabreichung von Tramadol-hydrochlorid und Calcium-Kanal Antagonisten eine Erhöhung der antinozizeptiven Wirkung von Tramadol bewirkt.

### Beispiel 2

Nach elektrischer Reizung der vorwiegend sensorischen Suralis-Nerven wurden die zum Gehirn aufsteigenden elektrischen Nervenimpulse im Ruckenmark der Ratte gemessen. Durch die unterschledlichen Leitungsgeschwindigkeiten ließen sich die Impulse der Aß- und Aγ-Fasern von denen der rein sensorischen C-Fasern trennen. Die Durchfuhrung der Experimente folgte im wesentlichen der von Jurna und Heinz beschriebenen Methode (Brain Res 171, 573-576 (1979). Es ist die durch Gabe der Wirkstoffe (einzeln oder geneinsam intravenos verabreicht) hervorgerufene prozentuale Anderung der C-Faseraktivität im Ruckenmark angegeben. Die Messung erfolgte 60 Minuten nach intravenöser Injektion der Substanzen.

| Substanz | % Änderung | Anzahl Experimente |
|---|---|---|
| Tramadol 14,6 mg/kg | - 1,9 + 10,3 | 3 |
| Diltiazem 2,15 mg/kg | 5,4 + 10,3 | 5 |
| Diltiazem 2,15 mg/kg + Tramadol 14,6 mg/kg | -18,6 + 12,1 | 5 |
| Verapamil 0,1 L mg/kg | -13, 3 ± 1,2 | 5 |
| Verapamil 0,1 mg/kg + Tramadol 14,6 mg/kg | -25,5 ± 10,7 | 5 |

Die Ergebnisse zeigen, daß Tramadol (als Hydrochlorid) zusammen mit einem Calcium-Kanal-Antagonisten in gemeinsamer Formulierung eine verstärkte antinozizeptive Wirkung hat.

## Patentansprüche

1. Kombinationspräparat, enthaltend Tramadol in Form der freien Base oder eines physiologisch verträglichen Salzes und mindestens einen Calcium-Kanal Antagonisten in getrennter oder gemeinsamer Formulierung.

2. Kombinationspraparat nach Anspruch 1, wobei Tramadol als physiologisch verträgliches Salz, vorzugsweise als Hydrochlorid, eingesetzt wird.

3. Kombinationspräparat nach einem der Anspruche 1 bis 2, wobei ein Calcium-Kanal Antagonist eingesetzt wird.

4. Kombinationspraparat nach einem der Anspruche 1 bis 3, wobei die Wirkstoffe als gemeinsame Formulierung vorliegen.

5. Kombinationspraparat nach einem der Ansprüche 1 bis 3, wobei die Wirkstoffe in getrennter Formulierung vorliegen.

6. Kombinationspraparat nach einem der Anspruche 1 bis 5, enthaltend 10 - 800 mg, vorzugsweise 20 - 400 mg Tramadol als Hydrochlorid oder der entsprechenden Menge eines anderen physiologisch verträglichen Salzes oder der freien Base.

7. Kombinationspräparat nach einem der Anspruche 1 bis 6, wobei der jeweilige Calcium-Kanal Antagonist in einer Dosierung von einem Viertel der minimalen Wirkdosis bis zur maximalen Tagesdosis, vorzugsweise einem Viertel der minimalen Wirkdosis bis zur Hälfte der maximalen Tagesdosis, enthalten ist.

8. Kombinationspraparat nach einem der Anspruche 1 bis 7 zur Anwendung bei der Therapie mittelschwerer bis schwerer, akuter oder chronischer Schmerzen.

## Claims

1. Combination preparation containing tramadol in the form of the free base or of a physiologically acceptable salt and at least one calcium channel antagonist in separate formulations or a joint formulation.

2. Combination preparation according to Claim 1 wherein tramadol is used as a physiologically acceptable salt, preferably as the hydrochloride.

3. Combination preparation according to one of Claims 1 and 2 wherein a calcium channel antagonist is used.

4. Combination preparation according to one of Claims 1 to 3 wherein the active substances are present in a joint formulation.

5. Combination preparation according to one of Claims 1 to 3 wherein the active substances are present in separate formulations.

6. Combination preparation according to one of Claims 1 to 5 containing 10 - 800 mg, preferably 20 - 400 mg, of tramadol as the hydrochloride, or the corresponding amount of another physiologically acceptable salt or of the free base.

7. Combination preparation according to one of Claims 1 to 6 wherein the calcium channel antagonist in question is present in a dosage of a quarter of the minimum active dose to the maximum daily dose, preferably a quarter of the minimum active dose to half the maximum daily dose.

8. Combination preparation according to one of Claims 1 to 7 for use in the therapy of moderately severe to severe acute or chronic pain.

## Revendications

1. Association médicamenteuse, contenant du tramadol sous forme de la base libre ou d'un sel acceptable du point de vue physiologique et au moins un antagoniste du canal calcium dans une formulation séparée ou commune.

2. Association médicamenteuse suivant la revendication 1, dans laquelle le tramadol est utilisé sous forme d'un sel acceptable du point de vue physiologique, de préférence sous forme du chlorhydrate.

3. Association médicamenteuse suivant- l'une des revendications 1 et 2, dans laquelle un antagoniste du canal calcium est utilisé.

4. Association médicamenteuse suivant l'une des revendications 1 à 3, dans laquelle les substances actives sont présentes sous forme d'une formulation commune.

5. Association médicamenteuse suivant l'une des revendications 1 à 3, dans laquelle les substances actives sont présentes en formulation séparée.

6. Association médicamenteuse suivant l'une des revendications 1 à 5, contenant 10 à 800 mg, de préférence 20 à 400 mg de tramadol sous forme du chlorhydrate ou de la quantité correspondante d'un autre sel acceptable du point de vue physiologique ou de la base libre.

7. Association médicamenteuse suivant l'une des revendications 1 à 6, dans laquelle l'antagoniste du canal calcium concerné est présent à une dose allant du quart de la dose minimale active à la dose journalière maximale, de préférence du quart de la dose minimale active à la moitié de la dose journalière maximale.

8. Association médicamenteuse suivant l'une des revendications 1 à 7, destinée à être utilisée dans le traitement de douleurs modérées à intenses, aiguës ou chroniques.
